# EUROPEAN PATENT APPLICATION

(11) **EP 1 783 117 A1**
(43) Date of publication of application: **09.05.2007**
(21) Application number: 05110348.9
(22) Date of filing: 04.11.2005
(51) Int. Cl.: C07D 215/18

(54) **Process for the preparation of a leukotriene antagonist and intermediates thereof**

(71) Applicant: ESTEVE QUIMICA, S.A., 08024 Barcelona (ES)
(72) Inventor: Coppi, Laura Esteve Quimica S.A., 08024, Barcelona (ES); Bartra Sanmartí, Martí Esteve Quimica S.A., 08024, Barcelona (ES); Gasanz Guillén, Yolanda Esteve Quimica S.A., 08024, Barcelona (ES); Monsalvatje Llagostera, Montserrat Esteve, 08024, Barcelona (ES); Talavera Escasany, Pedro Esteve Quimica S.A., 08024, Barcelona (ES)
(74) Representative: Barlocci, Anna

(57) **Abstract**

It comprises a preparation process of Montelukast from an intermediate compound of formula (V), which is previously prepared by reaction of the corresponding sulfonate with 1-(mercaptomethyl)cyclopropyl)methanol. Compound (V) is reacted with a Grignard reactant to convert the ester group into a tertiary alcohol, followed by conversion of the primary alcohol into a sulfonate, substitution of the sulfonate group by a cyano group, and finally transforming the cyano compound to the carboxilic acid compound by a hydrolysis reaction to afford Montelukast. Montelukast can also be prepared by a hydrolysis reaction of the corresponding amide. It also comprises intermediate compounds useful in such preparation process.

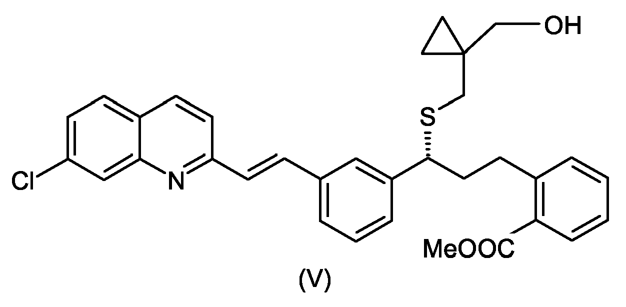

## Description

The present invention relates to a process for the preparation of Montelukast, as well as to some new intermediates useful in such preparation process.

### BACKGROUND ART

Montelukast, is the International Non-proprietary Name (INN) of1-[[[(1R)-1-[3-[(1E)-2-(7-chloro-2-quinolinyl)ethenyl]phenyl]-3-[2-(1-hydroxy-1-methylethyl)phenyl]propyl]sulfanyl]methyl]cyclopropaneacetic acid, and CAS No. 158966-92-8. Montelukast monosodium salt (CAS No 151767-02-1) is currently used in treatment of asthma, inflammation, angina, cerebral spasm, glomerular nephritis, hepatitis, endotoxemia, uveitis and allograft rejection.

The structure of Montelukast monosodium salt corresponds to formula (I):

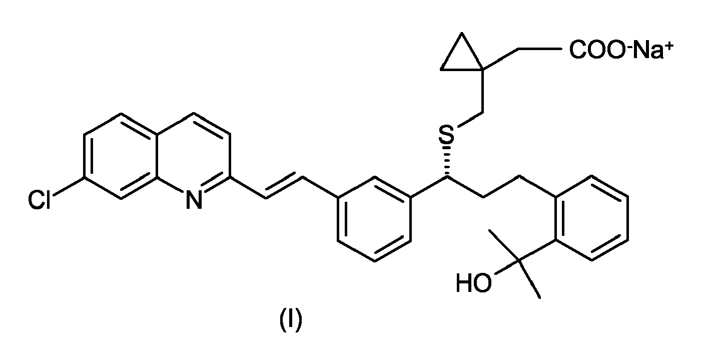

Different synthetic strategies for the preparation of Montelukast and its salts are known. EP 480.717 discloses certain substituted quinolone compounds including Montelukast sodium salt, methods for their preparation, and pharmaceutical compositions using these compounds. Several preparation processes of Montelukast sodium are reported in this document. Example 161 relates to the preparation of Montelukast sodium salt. According to this Example, preparation of Montelukast sodium salt proceeds through its corresponding methyl ester, whose preparation comprises sodium hydride or cesium carbonate assisted coupling of methyl-1-(mercaptomethyl)-cyclopropaneacetate with the protected mesylate (2-(2-(2-(3(S)-(3-(2-(7-chloro-2-quinolinyl)-ethenyl)phenyl)-3-(methanesulfonyloxy)propyl)phenyl)-2-propoxy)tetrahydropyran, generated *in situ.* The methyl ester thus obtained is hydrolyzed to the Montelukast free acid which is then converted directly to the sodium salt. This process is not particularly suitable for large scale production because it requires tedious chromatographic purification of the methyl ester intermediate and/or the final product, and yields of intermediates and final product are low. Another procedure for the preparation of Montelukast sodium salt comprises the reaction of the thioacetate with the following formula: 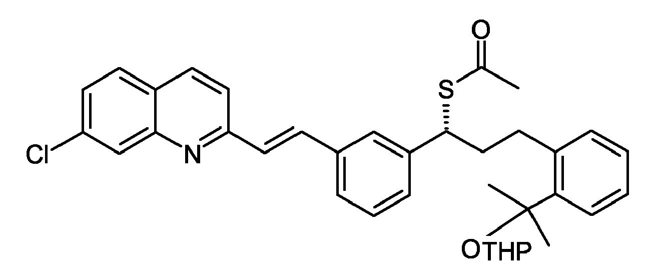 with hydrazine or an alkoxide, followed by reaction with an appropriate 1-substituted cyclopropyl acetate, followed by deprotection of the tertiary alcohol to afford Montelukast.

EP 737.186 relates to a process for the preparation of Montelukast or its salts thereof, which comprises reacting the dilithium dianion of 1-(mercaptomethyl)cyclopropaneacetic acid with the corresponding mesylate alcohol ((2-(2-(2-(3(S)-(3-(2-(7-chloro-2-quinolinyl)-ethenyl)phenyl)-3-(methanesulfonyloxy)propyl)phenyl)-2-propanol), to obtain Montelukast, which is further converted to the corresponding sodium salt via dicyclohexyl amine salt.

CN 1.420.113 A relates to a process for the preparation of Montelukast or its sodium salt thereof, which comprises the reaction of the thioacetic acid ester with the following formula: 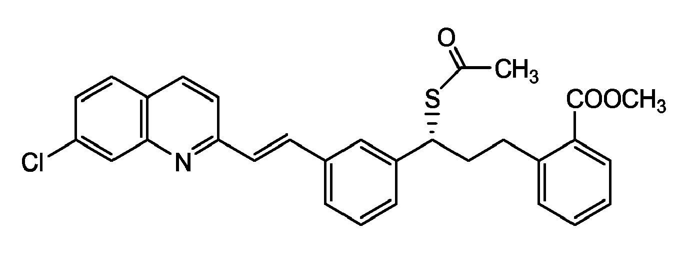 and methyl magnesium iodide to afford the thiol alcohol with the following formula:

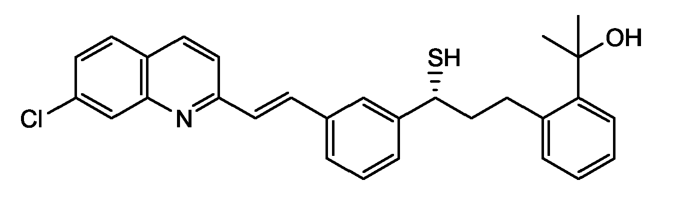

Montelukast is synthesized by reaction between the mentioned above thiol alcohol intermediate and appropriate 1-halo substituted cyclopropyl acetate, followed by hydrolysis of the ester group.

Finally, US 2005107612 describes a process for the preparation of Montelukast or a salt thereof, which comprises reacting a late intermediate compound which is 2-[1-[1-R-3-[2-(7 chloro quinolin-2-yl) vinyl [phenyl]-3-[2-methoxy carbonyl phenyl] propyl sulfonyl methyl] cyclo propyl] acetic acid or a salt thereof with methyl magnesium chloride or methyl magnesium bromide.

Although certain processes for the preparation of Montelukast are known, there continue being a need for new processes for the preparation of Montelukast and its salts.

### SUMMARY OF THE INVENTION

Inventors have found a new efficient preparation process of Montelukast from new intermediate compounds, which proceeds with high yields and without the need of chromatographic purifications.

Thus, according to one aspect of the present invention, it is provided a preparation process of Montelukast (I), or a pharmaceutically acceptable salt, or a solvate thereof, including a hydrate, comprising submitting the compound of formula (II) wherein R₁ is a radical selected from -CN and -CONH₂ to a hydrolysis reaction.

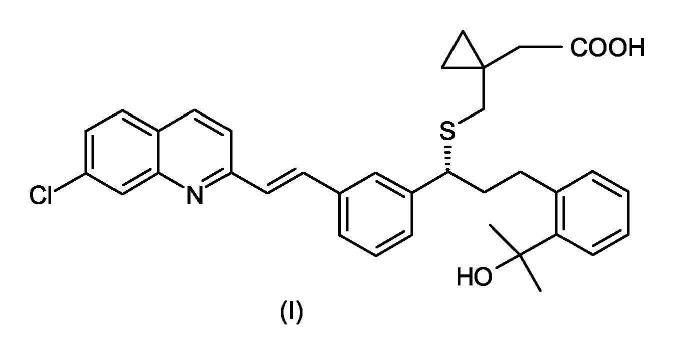

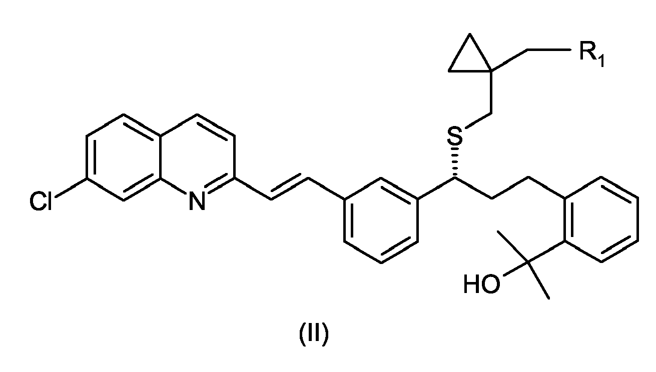

Compound of formula (II) with R₁ = -CONH₂, can be prepared from the compound of formula (II) with R₁ = -CN by hydrolisis. Compound of formula (II) with R₁=CN is obtained by reaction of a compound of formula (III) where R₂ is a radical selected from the group consisting of (C₁-C₄)-alkyl, phenyl, and phenyl mono- or disubstituted by a (C₁-C₄)-alkyl radical, with an alkaline metal cyanide, causing the substitution of the sulfonate group (-OSO₂-R₂) by the cyano group (-CN).

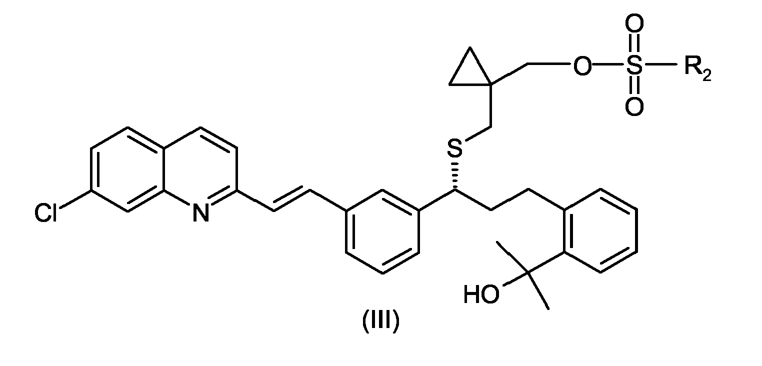

The compound of formula (III), is prepared by reaction of the alcohol of formula (IV) with a sulphonyl chloride of formula Cl-SO₂-R₂, wherein R₂ has the same meaning mentioned above.

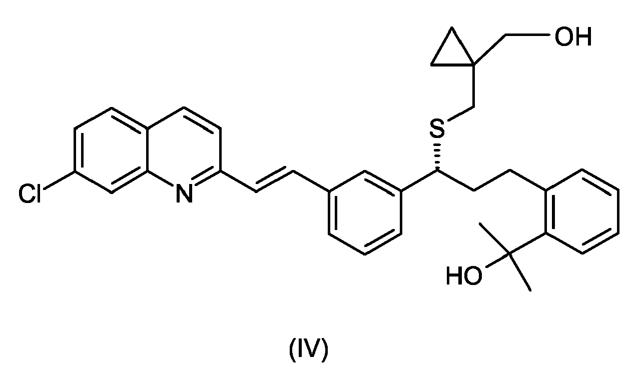

The compound of formula (IV) is prepared by reaction of the compound of formula (V) with a Grignard reagent selected from the group consisting of methyl lithium and a methyl magnesium halide, optionally in the presence of cerium chloride.

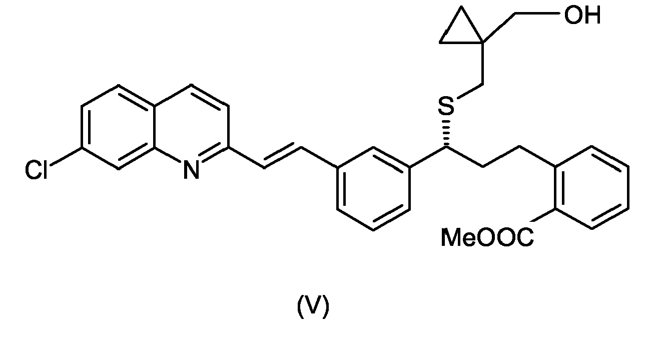

The compound of formula (V) is prepared by reaction of compound of formula (VI) where R₃ is a radical selected from the group consisting of (C₁-C₄)-alkyl, phenyl, and phenyl mono- or disubstituted by (C₁-C₄)-alkyl radicals, with a compound of formula (VII), in the presence of a base selected from the group consisting of cesium carbonate, sodium hydroxide, and lithium bis(trimethylsilyl)amide.

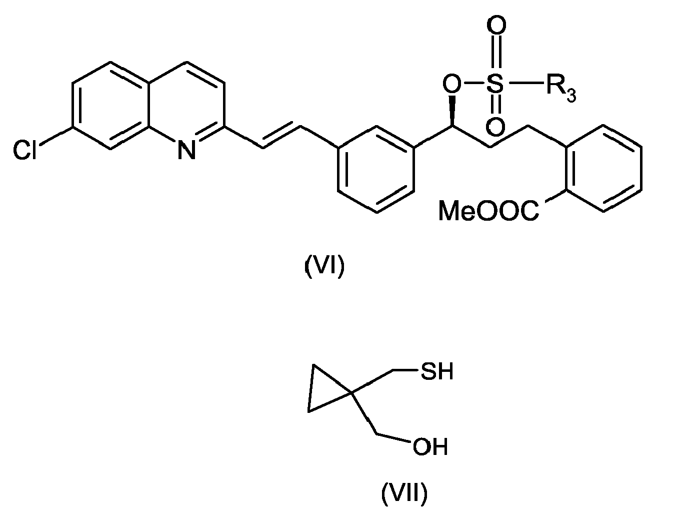

The above defined compounds of formula (II), (III), (IV) and (V) are new and form also part of the invention. Thus, another aspect of the present invention is to provide new intermediate compounds for the preparation of Montelukast, in particular those of formula (II), (III), (IV) and (V).

The process of the present invention is particularly advantageous in its practical industrial realization because is cost effective and suited for scale up. Unlike other known processes for the preparation of Montelukast, this process avoids intramolecular cyclizations of the intermediate compounds. Thus, all intermediates are formed cleanly, and therefore chromatographic separations were not required. Furthermore, the final product is obtained in high chemical and optical purity and with high yields.

### DETAILED DESCRIPTION OF THE INVENTION

As it is described above, the last step of the process is a hydrolysis reaction of the compound of formula (II) to afford Montelukast. Preferably, the hydrolysis of the compound of formula (II) is carried out with a base. Preferably the base is an alkaline metal hydroxide or an alkaline earth metal hydroxide. More preferably, the base is sodium hydroxide, potassium hydroxide or lithium hydroxide. The most preferred one is sodium hydroxide. The reaction can be carried out in different solvent systems. Preferably the solvent system is a solvent mixture comprising C₁-C₄ alcohol and water. Preferably the C₁-C₄ alcohol is ethanol or isopropanol. The most preferred one is ethanol. Tipically, the reaction is carried out at reflux temperature and it is essentially complete within 24 hours. Other suitable solvent system can be a two-phase solvent mixture comprising water and a suitable organic solvent non-miscible in water or sparingly miscible in water, optionally in the presence of a phase transfer catalyst. Preferably the organic solvent is a (C₆-C₈)-aromatic hydrocarbon. The most preferred one is toluene Suitable phase transfer catalyst include for instance, an ammonium quaternarium salt such as tetrabutylamonium bromide, tri-C₈-C₁₀-alkylmethylammonium chlorides, methyltrioctilammonium chloride or hexadeciltrimethylammonium chloride. The hydrolysis can be carried out at a temperature comprised between room temperature and reflux temperature. Tipically, the reaction is carried out at 120 °C and it is essentially complete within about seven days.

The isolation of crude Montelukast is accomplished by diluting de crude with toluene and washing the solution with aqueous acid acetic and susbsequently with water at room temperature, followed by evaporation of the solvent. The obtained Montelukast can be purified by several methods, for instance, by aqueous acid-base extractions or by recristallization.

Compound of formula (II) with R₁ = -CONH₂, can be prepared from the compound of formula (II) with R₁ = -CN by hydrolisis. For instance, hydrolisis can be achieved using mild conditions of hydrolysis, or by shortening the reaction time.The conditions to carry out the hydrolysis may be easily determined by the person skilled in the art by routine tests. For example, the hydrolysis can be carried out using the reacting conditions described above for carrying out the hydrolysis of the cyano compound to Montelukast but reducting the reaction time, as it illustrated in Example 9, followed by the isolation of the amide compound of formula (II).

Montelukast free acid obtained by the process of the present invention may be converted into pharmaceutically acceptable salts, and salts may be converted into free acid compounds, by known methods described in the art. It is also possible to isolate a salt of Montelukast from the hydrolysis reaction, i.e. without isolating the montelukast free acid.

As previously described above, the cyano compound of formula (II) is obtained from the sulfonate of formula (III). The conversion is carried out by reaction of the compound (III) with an alkaline metal cyanide such as sodium or potassium cyanide, in an appropriate solvent such as dimethylformamide, dimethylsufoxide, ethyl acetate or acetonitrile, and at a temperature comprised between 0°C and reflux temperature. Preferably, the reaction is carried out at about 60 °C.

In the present invention, preferred sulfonate compounds of formula (III) are those where the sulfonate (III) is a mesylate (R₂= methyl), a besylate (R₂= phenyl) or a tosylate (R₂= 4-methylphenyl). The most preferred sulfonate (III) is the mesylate.

The sulfonate compounds are prepared from the alcohol of formula (IV) by reaction with the corresponding sulfonyl chloride. This reaction is carried out in an appropriate solvent and in the presence of a tertiary amine, at a temperature comprised between -20 °C and room temperature. Preferably, the reaction is carried out at low temperatures. Common solvents for the reaction include chlorine-containing solvents such as methylene chloride or 1,2-dichloroethylene, aromatic hydrocarbons such as toluene or xylene, and dimethylformamide. Examples of suitable tertiary amines are diisopropylethylamine and triethylamine.

The preparation of the alcohol of formula (IV) from the compound of formula (V) is carried out by reaction with a methyl magnesium halide such as methyl magnesium chloride or methyl magnesium bromide, optionally in the presence of cerium chloride, or by reaction with methyl lithium, in the presence of a suitable solvent. Examples of suitable solvents includes an ether such as tetrahydrofurane, or an aromatic hydrocarbon such as toluene or xylene, or mixtures of them. Preferably the reaction is carried out at a temperature comprised between -78 °C and 20 °C, more preferably, at -20 °C.

The compound of formula (V) is prepared from the known compound of formula (VI), which is reacted with the compound of formula (VII) in a suitable solvent such as dimethylformamide, dimethylsulfoxide, dichloromethane, toluene or tetrahydrofurane, and in the presence of a base such as cesium carbonate, sodium hydroxide and lithium bis(trimethylsilyl)amide. Preferably, the reaction is carried out at a temperature comprised between -10 °C and 60 °C, more preferably, at a 0-5°C.

The compound of formula (VI) may be prepared according to a known process described in US 2005107612. The process comprises the reaction of the methyl-2-((S)-3-(3-((E)-2-(7-chloroquinolin-2-yl)vinyl)phenyl)-3-hydroxypropyl)benzoate of Formula (VIII) with methane sulfonyl chloride or toluene sulfonyl chloride to form the methyl-2-((S)-3-(3-(E)-(2-(7-chloroquinolin-2-yl)vinyl)phenyl)-3-[(methylsulfonyl)oxy]propyl)benzoate or the corresponding tosylate. Analogously, the besylate of formula (VI) can be obtained from the corresponding benzene sulfonyl chloride.

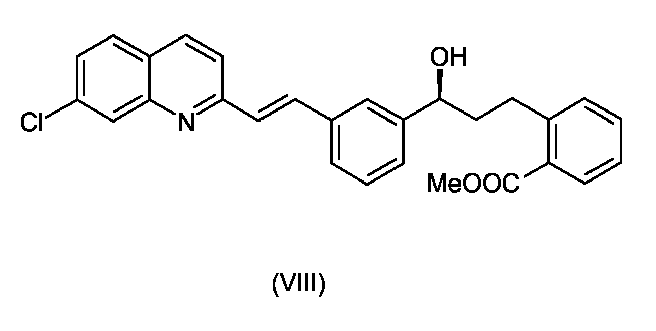

The compound of formula (VII) can be prepared from 5,7-dioxa-6-thiaspiro[2.5]octane 6-oxide according to the process summarized in Scheme 1.

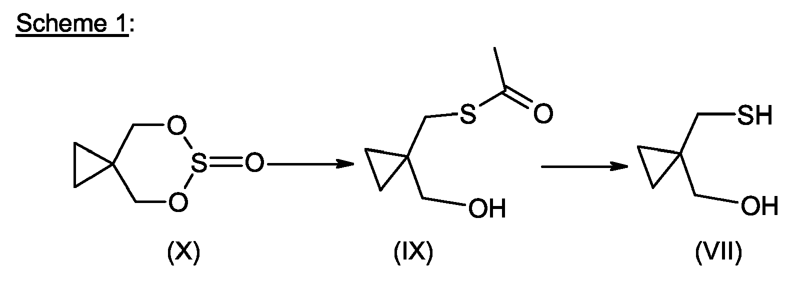

The preparation process of the compound of formula (VII) involves the reaction of the compound of formula (X) with potassium ethanethioate to give the compound of formula (IX). Tipically, such reaction is carried out with an excess of potassium ethanethiolate in an appropriate solvent. Examples of suitable solvents include dimethylsulfoxide, dimethylformamide, ethyl acetate, acetonitrile or mixtures thereof. Preferably the reaction is carried out at a temperature comprised between room temperature and 70°C. After the isolation of the compound of formula (IX), it can be hydrolized using an acid or a basic catalyst to give the compound of formula (VII). Examples of suitable acids are hydrochloric acid, sulfuric acid, acetic acid and formic acid. Examples of suitable bases are hydroxides, carbonates and alcoxide of an alkaline or an earth alkaline metal. Example of suitable solvents are (C₁-C₆)-alcohols, (C₆-C₈)-aromatic hydrocarbons, dimethylformamide, dimethylsulfoxide, or mixtures of them.

The best conditions to carry out the process of the present invention vary according to the parameters considered by a person skilled in the art, such as the starting materials, molar ratio, temperature, and similar. Such reaction conditions may be easily determined by a person skilled in the art by routine tests, and with the teaching of the examples included in this document.

Throughout the description and claims the word "comprise" and variations of the word, are not intended to exclude other technical features, additives, components, or steps. The abstract of this application is incorporated herein as reference. Additional objects, advantages and features of the invention will become apparent to those skilled in the art upon examination of the description or may be learned by practice of the invention. The following examples are provided by way of illustration, and they are not intended to be limiting of the present invention.

### EXAMPLES

### Example 1: Preparation of S-(1-(hydroxymethyl)cyclopropyl)methyl ethanethioate (IX)

200 g of 5,7-dioxa-6-thiaspiro[2.5]octane 6-oxide were dissolved in 1.2 l of dimethyl sulfoxide and 308 g of potassium ethanethioate were poured to the solution. Then, the suspension was heated at 40°C for 5. Once the reaction was completed, a combination of 3.6 l of ethyl acetate and 3.6 l of water was added. The organic phase was separated, washed with water and concentrated to dryness. 200 g of S-(1-(hydroxymethyl)cyclopropyl)methyl ethanethioate were recovered. Yield: 78% corrected by GC. ¹H NMR (400 MHz, CDCl₃): 3.45 (2H, d, J: 6.4 Hz); 3.01 (2H, s); 2.53 (OH, broad triplet, J: 6.4 Hz); 2.39 (3H, s); 0.54 (4H, m)

### Example 2: Preparation of (1-(mercaptomethyl)cyclopropyl)methanol (VII)

200 g of S-(1-(hydroxymethyl)cyclopropyl)methyl ethanethioate were dissolved in 2 l of methanol. Then, 111 ml of concentrated HCl were added under a nitrogen blanket and the solution was stirred at room temperature for 10. The solvent was distilled off and the residue was re-dissolved in 1.5 l of dimethylformamide to be used in the preparation of methyl 2-((R)-3-(3-((E)-2-(7-chloroquinolin-2-yl)vinyl)phenyl)-3-(((1-(hydroxymethyl) cyclopropyl)methyl)sulfanyl)propyl)benzoate. Yield: 100%. ¹H NMR (400 MHz, CDCl₃): 3.57 (2H, s); 2.63 (2H, d, J: 8.0 Hz); 2.45 (OH, broad signal,); 1.43 (SH, t, J: 8.0 Hz); 0.52 (4H, m)

### Example 3: Preparation of methyl 2-((S)-3-(3-((E)-2-(7-chloroquinolin-2-yl)vinyl)phenyl)-3-[(methylsulfonyl)oxy]propyl)benzoate (VI)

12.1 ml de diisopropylethylamine were poured to a stirred solution of 24.5 g of methyl 2-((S)-3-(3-((E)-2-(7-chloroquinolin-2-yl)vinyl)phenyl)-3-hydroxypropyl)benzoate in 120 ml of dichloromethane. The mixture is cooled to -20 °C and 5 ml of mesyl chloride were added slowly. Once the reaction was completed, the crude solution was successively washed with 120 ml of an aqueous 10% NaHCO₃ solution and 120 ml of water. Finally, the solvent was distilled off to obtain 29 g of the title compound. The product was re-dissolved in 290 ml of dimethylformamide to be used as a solution in the next step. Yield: 100%.

### Example 4: Preparation of methyl 2-((R)-3-(3-((E)-2-(7-chloroquinolin-2-yl)vinyl)phenyl)-3-(((1-(hydroxymethyl)cyclopropyl)methyl)sulfanyl)propyl) benzoate (V)

A solution of 19 g of (1-(mercaptomethyl)cyclopropyl)methanol in 190 ml of dimethylformamide was cooled to 0 °C. Then, 52 g of Cs₂CO₃ were added in one portion. After 10 min, the outcome solution from the previous example was added and the suspension was maintained at the same temperature for 18 hours. Finally, a mix of 380 ml of ethyl acetate and 380 ml of water was poured to the solution crude. The organic phase was washed with 380 ml of water and concentrated to dryness. 30 g of brown oil were recovered. Yield: 86%, corrected after HPLC analysis. ¹H NMR (400 MHz, DMSO-d₆): 8.38 (1 H, d, J: 8.4 Hz); 8.00-7.26 (14H, m); 4.50 (OH, t, J: 5.4 Hz); 3.92 (1 H, t, 7.2 Hz); 3.76 (3H, s); 3.33 (1H, dd, J: 11.0 Hz, 5.4 Hz); 3.25 (1H, dd, J: 11.0 Hz, 5.4 Hz); 2.98-2.88 (1H, m); 2.83-2.74 (1 H, m); 2.52 (1H, d, J: 12.7 Hz); 2.39 (1H, d, J: 12.7 Hz); 2.10 (2H, m); 0.46-0.18 (4H, m)

### Example 5: Preparation of 2-(2-((R)-3-(3-((E)-2-(7-chloroquinolin-2-yl)vinyl)phenyl)-3-(((1-(hydroxymethyl)cyclopropyl)methyl)sulfanyl)propyl) phenyl)propan-2-ol (IV)

The product obtained in the previous example was dissolved in 300 ml of tetrahydrofuran. The mixture was cooled to 0 °C and 307 ml of 1.4M solution of methylmagnesium bromide were added under a nitrogen blanket. The reaction was maintained at 0 °C for 18 hours. Then, 400 ml of 2M aqueous solution of acetic acid were added slowly, followed of 400 ml of ethyl acetate. The organic phase was successively washed with 400 ml of an aqueous 10% NaHCO₃ solution and 400 ml of water. Finally, the solvent was distilled off to yield 30 g of crude. Yield: 81% corrected after HPLC analysis. ¹H NMR (400 MHz, DMSO-d₆): 8.39 (1H, d, J: 8.6 Hz); 8.00-7.06 (14H, m); 4.91 (OH, s); 4.51 (OH, t, J: 5.5 Hz); 3.99 (1 H, t, 7.3 Hz); 3.34 (1H, dd, J: 11.0 Hz, 5.5 Hz); 3.26 (1H, dd, J: 11.0 Hz, 5.5 Hz); 3.05 (1H, m); 2.74 (1H, m); 2.54 (1H, d, J: 12.7 Hz); 2.41 (1H, d, J: 12.7 Hz); 2.15 (2H, m); 1.44 (6H, s); 0.45-0.36 (2H, m); 0.33-0.22 (2H, m).

### Example 6: Preparation of 2-(2-((R)-3-(3-((E)-2-(7-chloroquinolin-2-yl)vinyl)phenyl)-3-(((1-(hydroxymethyl)cyclopropyl)methyl)sulfanyl)propyl) phenyl)propan-2-ol (IV)

1.3 g of anhydrous cerium chloride were stirred in 10 ml of dry tetrahydrofuran for 18 hours at room temperature. Then, the suspension was cooled to -20 °C and 1.5 ml of a 3M solution of methylmagnesium chloride were added under a nitrogen blanket. After a period of time, a 2.5 ml tetrahydrofuran solution of 0.5 g of the product obtained in example 4 was added. The mixture was stirred for 18 hours at the same temperature and 10 ml of 2M aqueous solution of acetic acid were added slowly, followed of 10 ml of ethyl acetate. The organic phase was successively washed with 10 ml of an aqueous 10% NaHCO₃ solution and 10 ml of water. Finally, the solvent was distilled off to yield 0.5 g of crude. Yield: 80% corrected after HPLC analysis.

### Example 7: Preparation of (R,E)-(1-((1-(3-(2-(7-chloroquinolin-2-yl)vinyl)phenyl)-3-(2-(2-hydroxypropan-2-yl)phenyl)propyl)sulfanyl)methyl) cyclopropyl)methyl methanesulfonate ((III) with R₂= CH₃)

6.5 ml de diisopropylethylamine were poured to a stirred solution of 16 g of 2-(2-((R)-3-(3-((E)-2-(7-chloroquinolin-2-yl)vinyl)phenyl)-3-(((1-(hydroxymethyl)cyclopropyl)methyl)sulfanyl)propyl)phenyl)propan-2-ol in 160 ml of dichloromethane. The mixture was cooled to -20 °C and 2.7 ml of mesyl chloride were added slowly. The mixture was stirred at the same temperature for one hour and then, it was washed twice with 300 ml of water. The solvent was distilled off and 18 g of crude were recovered. Yield: 100%. ¹H NMR (400 MHz, DMSO-d6): 8.41 (1H, d, J: 9.6 Hz); 8.02-7.03 (14H, m); 4.91 (OH, s); 4.12 (2 H, m); 4.02 (1H, t, J: 7.1 Hz); 3.12 (3H, s); 3.09-3.02 (1H, m); 2.78-2.71 (1H, m); 2.54 (1H, d, J: 13.2 Hz); 2.46 (1H, d, J: 13.2 Hz); 2.15 (2H, m); 1.43 (3H, s); 1.42 (3H, s); 0.68-0.45 (4H, m)

### Example 8: Preparation of (R,E)-2-(1-((1-(3-(2-(7-chloroquinolin-2-yl)vinyl)phenyl)-3-(2-(2-hydroxypropan-2-yl)phenyl)propyl)sulfanyl)methyl) cyclopropyl)acetonitrile ((II) with R₁=CN)

The crude obtained in the previous example was dissolved in 180 ml of dimethylformamide and 2.1 g of sodium cyanide were added in one portion to the solution. After 18 hours of stirring at 60 °C, 270 ml of ethyl acetate was poured into the mixture previously warmed to room temperature. Then, two washes with 270 ml of water were carried out and the solvent was distilled off to afford 14 g of crude. Yield: 75% corrected after HPLC analysis. ¹H NMR (400 MHz, DMSO-d₆): 8.39 (1H, d, J: 8.6 Hz); 8.00-7.05 (14H, m); 4.90 (OH, s); 4.04 (1H, t, J: 7.2 Hz); 3.06 (1H, m); 2.76 (1H, m); 2.67 (1H, m); 2.33 (1H, m); 2.30 (2H, s); 2.13 (2H, m); 1.43 (6H, s); 0.59-0.43 (4H, m)..

### Example 9: Preparation of (R,E)-2-(1-((1-(3-(2-(7-chloroquinolin-2-yl)vinyl)phenyl)-3-(2-(2-hydroxypropan-2-yl)phenyl)propyl) sulfanyl)methyl)cyclopropyl)acetamide ((II) with R₁=CONH₂)

1.8 g of crude obtained in example 8 were dissolved in 3.6 ml of toluene. Then, 3.6 ml of water was added followed of 14.7 g of sodium hydroxide and 0.44 g of tetrabutylammonium bromide. The mixture was stirred at 120 °C for 30 hours. After this period of time, 10 ml of toluene and 10 ml of water were added to the reaction solution previously cooled to room temperature. The organic phase was separated and washed with 10 ml of water. Finally, the solvent was distilled off and the crude was purified by standard methods in order to recover 77 mg of the title compound. Yield: 6%.¹H NMR (400 MHz, DMSO-d₆): 8.41 (1H, d, J: 8.6 Hz); 8.03-7.07 (15H, m); 6.74 (1H, s); 4.91 (OH, s); 4.02 (1H, t, J: 7.3 Hz); 3.04 (1H, m); 2.75 (1H, m); 2.57 (1H, d, J: 13.3 Hz); 2.45 (1H, d, J: 13.3 Hz); 2.16 (4H, m); 1.43 (6H, s); 0.50-0.27 (4H, m).

### Example 10: Preparation of 2-(1-((((R)-1-(3-((E)-2-(7-chloroquinolin-2-yl)vinyl)phenyl)-3-(2-(2-hydroxypropan-2-yl)phenyl)propyl)sulfanyl)methyl) cyclopropyl)acetic acid (Montelukast free acid)

5.2 g of crude obtained in example 8 were dissolved in 10 ml of toluene. Then, 10 ml of water was added followed of 14.7 g of sodium hydroxide and 0.44 g of tetrabutylammonium bromide. The mixture was stirred at 120 °C for 7 days. After this period of time, 10 ml of toluene and 10 ml of water were added to the reaction solution previously cooled to room temperature. The organic phase was separated and successively washed with 10 ml of acetic acid and 10 ml of water. Finally, the solvent was distilled off to recover 4.6 g of product, that can be purified by standard methods if it is necessary. Yield: 83%.¹H NMR (400 MHz, DMSO-d₆): 12.02 (1H, s); 8.39 (1H, d, J: 8.6 Hz); 8.01-7.04 (14H, m); 4.90 (OH, s); 4.00 (1H, t, J: 7.3 Hz); 3.05 (1H, m); 2.76 (1H, m); 2.56 (1H, d, J: 12.9 Hz); 2.47 (1H, d, J: 12.9 Hz); 2.32 (2H, s); 2.16 (2H, m); 1.44 (6H, s); 0.52-0.33 (4H, m). IR (KBr) = 3573.1, 3436.9, 2919.2, 1716.9, 1608.9, 1499.9, 1408.8, 1315.7, 1223.9, 1201.6, 1173.0, 1148.0, 1134.9, 1074.8, 965.9, 950.8, 933.2, 863.6, 842.7, 766.0 cm⁻¹.

### Example 11: Preparation of 2-(1-((((R)-1-(3-((E)-2-(7-chloroquinolin-2-yl)vinyl)phenyl)-3-(2-(2-hydroxypropan-2-yl)phenyl)propyl)sulfanyl)methyl) cyclopropyl)acetic acid (Montelukast free acid)

1.05 g of sodium hydroxide were poured to a solution of 1 g of (R,E)-2-(1-((1-(3-(2-(7-chloroquinolin-2-yl)vinyl)phenyl)-3-(2-(2-hydroxypropan-2-yl)phenyl)propylthio)methyl)cyclopropyl)acetonitrile in 5 ml of a mixture of ethanol:water (96:4, v/v). The outcome suspension was stirred at reflux temperature for 15 hours. After this period of time, the reaction mixture was cooled down to room temperature and diluted with 10 ml of toluene. Then, 20 ml of a 2M aqueous solution of acetic acid were added slowly and the organic layer was separated and washed twice with 10 ml of water. Finally, the solvent was distilled off to recover 0.9 g of product that can be purified by standard methods if it is necessary. Yield: 91%.

### Example 12: Preparation of sodium 2-(1-((((R)-1-(3-((E)-2-(7-chloroquinolin-2-yl)vinyl)phenyl)-3-(2-(2-hydroxypropan-2-yl)phenyl)propyl)sulfanyl)methyl) cyclopropyl) acetate (Montelukast sodium)

2.6 g of Montelukast free acid were dissolved in 26 ml of toluene and 8.9 ml of 0.5M NaOH solution in methanol were added slowly at room temperature. The mixture was stirred for 1 hour and the solvent was removed under vacuum to obtain a residue. Then, heptane (24 ml) was added over 30 min to a well stirred solution of the residue in 4 ml of ethyl acetate at room temperature. Two hours after the addition, an off white solid was filtered off under a nitrogen atmosphere and washed with 5 ml of heptane. The wet product was dried under vacuum at 70-80 °C for 2 days to yield 2.7 g of amorphous solid form of Montelukast sodium. Yield: 100%. ¹H NMR (400 MHz, DMSO-d₆): 8.38 (1H, d, J: 8.6 Hz); 8.02-7.04 (14H, m); 5.19 (OH, s); 4.01 (1H, t, J: 7.2 Hz); 3.08 (1H, m); 2.72 (1H, m); 2.69 (1H, d, J: 12.4 Hz); 2.54 (1H, d, J: 12.4 Hz); 2.22 (1H, m); 2.10 (1H, d, J: 14.2 Hz); 2.10 (1H, m); 1.96 (1H, d, J: 14.2 Hz); 1.45 (3H, s); 1.44 (3H, s); 0.46-0.32 (2H, m); 0.28-0.15 (2H, m).

## Claims

1. A preparation process of Montelukast (I), or a pharmaceutically acceptable salt, or a solvate thereof, including a hydrate, comprising submitting the compound of formula (II) wherein R₁ is a radical selected from -CN and-CONH₂ to a hydrolysis reaction.

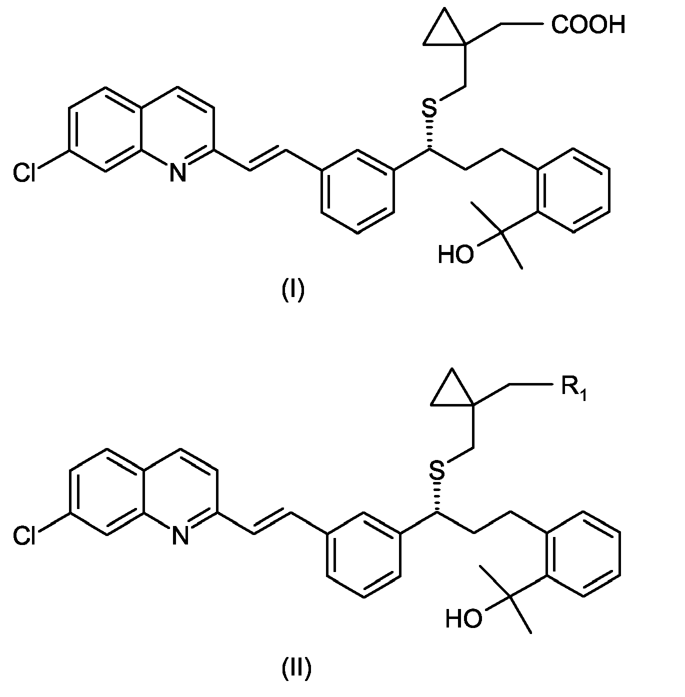

2. The preparation process according to claim 1, wherein the hydrolysis is carried out with a base.

3. The preparation process according to claim 2, wherein the base is selected from an alkaline metal hydroxide and alkaline earth metal hydroxide.

4. The preparation process according to claim 3, wherein the base is sodium hydroxide.

5. The preparation process according to any of claims 1-4, wherein the hydrolysis is carried out into a mixture comprising water and an organic solvent, optionally in the presence of a phase transfer catalyst.

6. The preparation process according to claim 5, wherein the phase transfer catalyst is an ammonium quaternarium salt selected from the group consisting of tri-C₈₋₁₀-alkylmethylammonium chlorides, tetrabutylamonium bromide, hexadeciltrimethylammonium chloride and methyltrioctilammonium chloride.

7. The preparation process according to any of claims 5-6, wherein the organic solvent is selected from the group consisting of a C₁-C₄ alcohol and toluene.

8. The preparation process according to any of claims 1-7, wherein the compound of formula (II) with R₁= CONH₂ is prepared by hydrolysis of the compound of formula (II) with R₁= CN.

9. The preparation process according to any of claims 1-8, wherein the compound of formula (II) with R₁= CN is prepared by reaction of a compound of formula (III) wherein R₂ is a radical selected from the group consisting of (C₁-C₄)-alkyl, phenyl, and phenyl mono- or disubstituted by a (C₁-C₄)-alkyl radical, with an alkaline metal cyanide, causing the substitution of the sulfonate group (-OSO₂-R₂) by the cyano group (-CN).

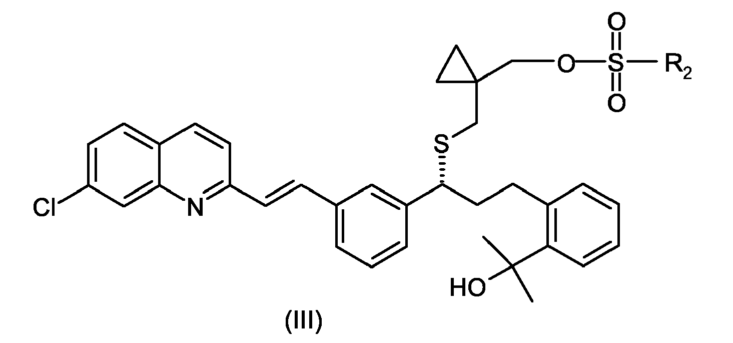

10. The preparation process according to claim 9, wherein R₂ is selected from the group consisting of methyl, phenyl, and 4-methylphenyl.

11. The preparation process according to claim 10, wherein R₂ is methyl.

12. The preparation process according to any of claims 9-11, wherein the compound of formula (III) is prepared by reaction of the alcohol of formula (IV) with a sulphonyl chloride of formula Cl-SO₂-R₂, wherein R₂ has the same meaning as in claims 8-10.

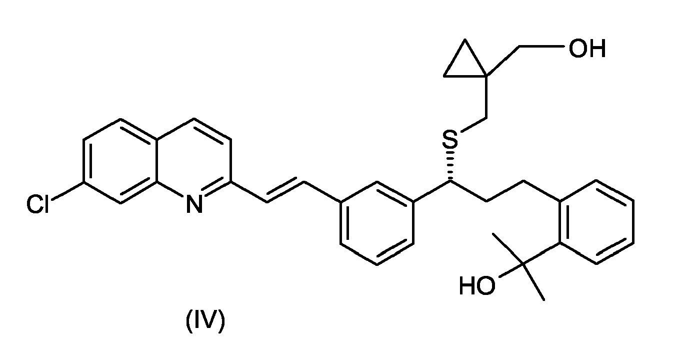

13. The preparation process according to claim 12, wherein the compound of formula (IV) is prepared by reaction of the compound of formula (V) with a Grignard reagent selected from methyl lithium and a methyl magnesium halide, optionally in the presence of cerium chloride.

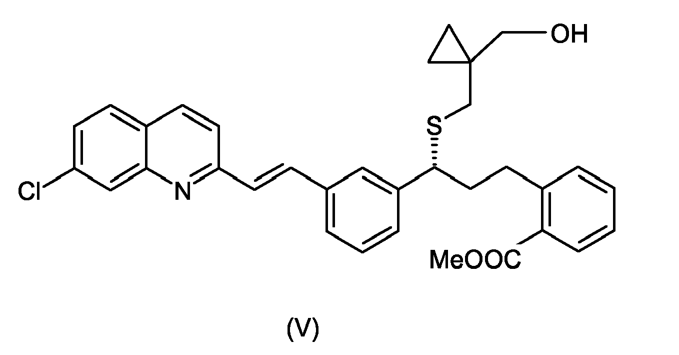

14. The preparation process according to claim 13, wherein the methyl magnesium halide is methyl magnesium chloride.

15. The preparation process according to any of claims 13-14, wherein the compound of formula (V) is prepared by reaction of compound of formula (VI) wherein R₃ is a radical selected from the group consisting of (C₁-C₄)-alkyl, phenyl, and phenyl mono- or disubstituted by (C₁-C₄)-alkyl radicals, with a compound of formula (VII), in the presence of a base.

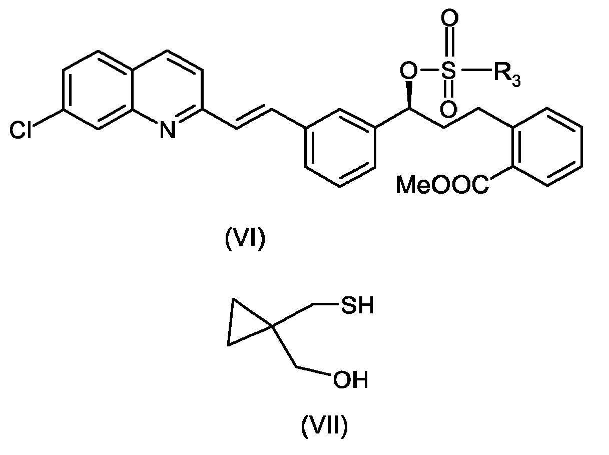

16. The preparation process according to claim 15, wherein R₃ is selected from the group consisting of methyl, phenyl, and 4-methylphenyl.

17. The preparation process according to claim 16, wherein R₃ is methyl.

18. The preparation process according to any of claims 15-17, wherein the base is selected from the group consisting of cesium carbonate, sodium hydroxide, and lithium bis(trimethylsilyl)amide.

19. The preparation process according to claim 18, wherein the base is cesium carbonate

20. A compound of formula (II), wherein R₁ is a radical selected from -CN and -CONH₂ .

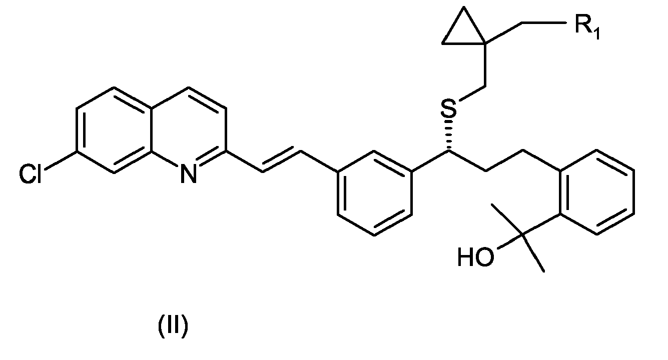

21. A compound of formula (III), wherein R₂ is a radical selected from the group consisting of (C₁-C₄)-alkyl, phenyl, and phenyl mono- or disubstituted by a (C₁-C₄)-alkyl radical.

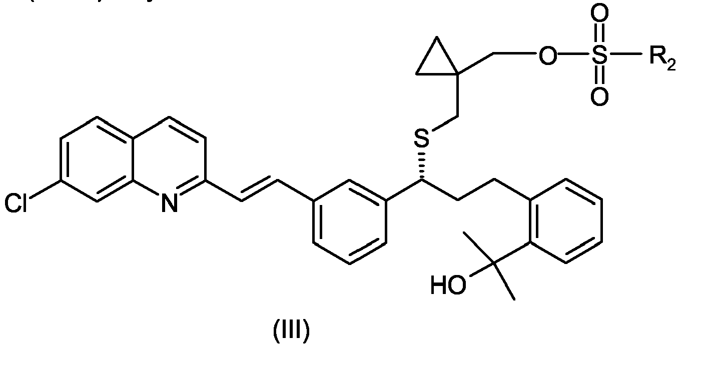

22. The compound according to claim 21, wherein R₂ is selected from methyl, phenyl, and 4-methylphenyl.

23. The compound according to claim 22, wherein R₂ is methyl.

24. A compound of formula (IV).

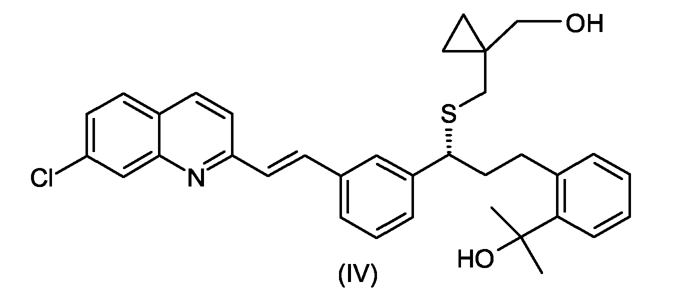

25. A compound of formula (V)
